# EUROPÄISCHE PATENTSCHRIFT

(11) **EP 3 442 982 B1**
(45) Veröffentlichungstag und Bekanntmachung des Hinweises auf die Patenterteilung: **01.07.2020**
(21) Anmeldenummer: 17721532.4
(22) Anmeldetag: 12.04.2017
(51) Int. Cl.: C07F 17/02, C07C 45/50, C07F 7/10

(54) **KATALYSATOR ZUR HYDROFORMYLIERUNG VON OLEFINEN UND DESSEN VERWENDUNG**
CATALYST FOR THE HYDROFORMYLATION OF OLEFINS, AND USE THEREOF
CATALYSEUR POUR L'HYDROFORMYLATION D'OLÉFINES ET UTILISATION CORRESPONDANTE

(30) Priorität: 14.04.2016 DE 102016206303
(43) Veröffentlichungstag der Anmeldung: 20.02.2019
(73) Patentinhaber: Technische Universität Berlin, 10623 Berlin (DE)
(72) Erfinder: DRIESS, Matthias, 10557 Berlin (DE); SCHOMÄCKER, Reinhard, 13589 Berlin (DE); BLOM, Burgert, 6211 SV Maastricht (NL); SCHMIDT, Marcel, 10587 Berlin (DE)
(74) Vertreter: Maikowski & Ninnemann Patentanwälte Partnerschaft mbB
(86) Internationale Anmeldenummer: PCT/EP2017/058800
(87) Internationale Veröffentlichungsnummer: WO 2017/178536

(56) Entgegenhaltungen:
- US-A1- 2009 299 099
- Dan E Hendriksen ET AL: "Selective Rhodium-Catalyzed Hydroformylation with the Tri-and Tetraphosphine Ligands (CH,), $i( CH2CH2PPh2)3,. Formation of Rh[Si(CH2CH2PPh2),](CO) via CH3-Si Bond Cleavage and Structure of This Rh( 1)-Si Bonded Complex", Organometallics, 1. Januar 1989 (1989-01-01), Seiten 1153-1157, XP55379461, Gefunden im Internet: URL:http://pubs.acs.org/doi/pdf/10.1021/om 00107a004

## Beschreibung

Die vorliegende Erfindung betrifft einen Katalysator für die Hydroformylierung von mindestens einem Olefin nach Anspruch 1, die Verwendung eines solchen Katalysators nach Anspruch 12 und ein Verfahren zur Hydroformylierung von Olefinen nach Anspruch 14.

### Beschreibung

Hydroformylierung ist ein bedeutender industrieller Prozess, bei welcher ein Olefin bzw. Alken mit Kohlenstoffmonoxid und Wasserstoff (Synthesegas) zu einem Aldehyd umgesetzt wird. Das Gesamtvolumen von durch Hydroformylierung produzierten Aldehyden beträgt derzeit durchschnittlich über 10 Mio. Tonnen im Jahr.

Für die Mehrzahl der Alkene können mehrere Produkte in Abhängigkeit von der Regioselektivität der Additionsreaktion gebildet werden. Das lineare und verzweigte Produkt werden im Allgemeinen als eine Mischung erhalten, wobei das lineare Produkt für industrielle Anwendungen bevorzugt ist. Die primär entstehenden Aldehyde werden meist zu Alkoholen hydriert, die als Weichmacher für PVC oder als Lösungsmittel dienen oder zu Polymeren weiterverarbeitet werden.

Es gibt derzeit zwei Hauptverfahren zur Hydroformylierung von Alkenen. Ein Verfahren involviert die Verwendung eines Kobaltkatalysators, welches im Allgemeinen für die Herstellung von höheren Aldehyden und Alkoholen mit einer Kettenlänge von C>5 angewendet wird. Da bei mittel- bzw. langkettigen Olefinen die Abtrennung des homogenen Katalysators schwierig ist, wird hierbei auf im Vergleich zu Rhodium billigere Kobaltkatalysatoren zurückgegriffen.

Im Gegensatz dazu werden Liganden-modifizierte Rhodiumkatalysatoren für kurzkettige Olefine verwendet, da hier der Katalysatorkomplex quantitativ abgetrennt werden kann (z.B. Ruhrchemie/Rhöne-Poulenc-Verfahren). Rhodium katalysierte Hydroformylierungen werden unter relativ milden Bedingungen durchgeführt und werden im Allgemeinen für die Hydroformylierung von Ethen und Propen, aber auch für die Umsetzung von 2-Propen-1-ol zu Butandiol verwendet. US 2009/299099 offenbart Katalysatoren zur Hydroformylierung von Olefinen, welche Phosphor-Liganden enthalten.

Es gibt einen Bedarf für selektivere und aktivere Katalysatoren zur Bildung von Aldehyden, welche auch für höhere Aldehyde anwendbar sind. Für die Steigerung der Aktivität und der Selektivität der Hydroformylierung kommt den in den Katalysatorkomplexen verwendeten Liganden spezielle Bedeutung zu.

Die Auswahl eines geeigneten Liganden ist entscheidend für die Aktivität und Selektivität der Hydroformylierung. Entsprechend stehen die Synthese und die Koordinationschemie von neuen Liganden im Fokus der Forschung. Vorteilhaft ist, dass die Anzahl von verschiedenen Liganden zur Verwendung in der Hydroformylierung schier unbegrenzt ist. Bisher wurden hauptsächlich Phosphor-, Stickstoff- oder Carbenliganden für die Hydroformylierung, insbesondere für die rhodiumkatalysierte Hydroformylierung verwendet. Phosphine, mit drei an das Phosphoratom gebundenen Alkylgruppen und Phophite, mit drei an das Phosphoratom gebundenen Alkoxygruppen sind von großem Interesse, wobei mit Phosphiten modifizierte Katalysatoren oft eine höhere Aktivität aufweisen. Auch die sterischen Eigenschaften der Liganden sind ein kritischer Parameter hinsichtlich der Regioselektivität der Hydroformylierung.

Aufgrund der großen wirtschaftlichen Bedeutung der Hydroformylierung gibt es aber nach wie vor einen Bedarf an Katalysatoren, die eine verbesserte Aktivität wie auch eine verbesserte Regioselektivität aufweisen.

Entsprechend liegt der vorliegenden Erfindung die Aufgabe zugrunde, neuartige Katalysatorsysteme für die Hydroformylierung von Olefinen zu entwickeln.

Diese Aufgabe wird mit einem Katalysator mit den Merkmalen des Anspruchs 1 gelöst.

Entsprechend wird ein Katalysator für die Hydroformylierung von mindestens einem Olefin bereitgestellt, welcher einen Liganden der allgemeinen Formel (I) 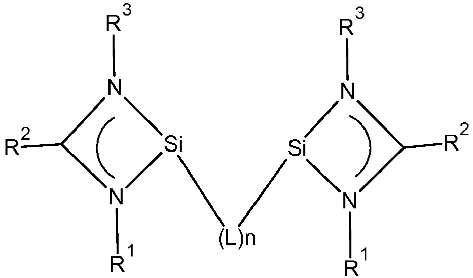 umfasst, wobei
- R¹, R², R³ ausgewählt sind aus der Gruppe umfassend substituiertes und nicht-substituiertes Alkyl, substituiertes und nicht-substituiertes Aryl, substituiertes und nicht-substituiertes Alkenyl, substituiertes und nicht-substituiertes Alkinyl, substituiertes und nicht-substituiertes Cycloalkyl, substituierte und nicht-substituierte Heterocyclen,
- wobei R¹, R² und R³ jeweils gleich oder verschieden voneinander sein können,
- L ausgewählt ist aus einer Gruppe umfassend einen Sandwich-Komplex, eine Sauerstoffgruppe, substituiertes und nicht-substituiertes Alkylen oder Heteroalkylen, substituiertes Aryl oder Heteroaryl, wobei Aryl und Heteroaryl jeweils mit mindestens zwei Heteroatome enthaltenden Gruppen substituiert sind und über die mindestens zwei Heteroatome der Substituenten mit dem Si gekoppelt sind,
- n = 1-10, bevorzugt 1-5, insbesondere bevorzugt 1, 2 oder 3 ist; und
- wobei der Ligand über die Si-Gruppe mit einem Metall M aus der Gruppe Vlllb des Periodensystems der Elemente gekoppelt ist.

Es wird somit ein Katalysator für die Hydroformylierung von Olefinen bereitgestellt, welcher N-heterozyklische Silylene (NHSi)-Liganden umfasst. Die vorliegend als Liganden verwendeten N-heterozyklischen Silylene sind schwerere Analoga der N-heterozyklischen Carbene (NHCs) und besitzen ein freies Elektronenpaar am Silizium, wodurch sie als Liganden für metallkatalysierte Reaktionen fungieren können. Die Besonderheit der N-heterozyklischen Silylene ist, dass sie sowohl als starker σ-Donor als auch als π-Akzeptor wirken, wodurch die elektronischen Eigenschaften am Metallzentrum des Katalysators entscheidend modifiziert werden können.

Die als Liganden zum Einsatz kommenden N-heterozyklischen Silylene sind zweizähnige (bidentate) Liganden. Aufgrund der Zweizähnigkeit wird nur ein Äquivalent des Liganden an das Metallzentrum gebunden und somit liegt eine freie Koordinationsstelle für die Bindung des Olefinsubstrates am katalytischen Zentrum vor.

In einer Ausführungsform wird Cobalt oder Rhodium als Metall M verwendet, wobei Rhodium für die katalysierte homogene Hydroformylierung von besonderer Bedeutung ist.

In einer Variante des vorliegenden Katalysators liegt der Linker L in Form eines Metallocen-Komplexes, d.h. einem Metallocen Komplex aus zwei jeweils substituierten oder nichtsubstituierten Cyclopentadien-Anionen gebunden an ein Metallzentrum, vor. Typische Metallocene sind Ferrocen, Titanocen Dicloride, Vanadocene Dichloride, wobei Ferrocen als Linker L besonders bevorzugt ist.

In einer anderen Variante des vorliegenden Katalysators liegt der Linker L in Form eines Phenyl- oder Pyridin-Ringes, die jeweils mit mindestens zwei ein Heteroatom (zur Kopplung an das Si) enthaltenden Gruppen substituiert sind, vor. Sauerstoff und Stickstoff sind dabei als Heteroatome bevorzugt. Phenyl- und Pyridin-Ring können darüber hinaus auch weitere Substituenten aufweisen.

Im Falle des Phenyl-Ringes als Linker L kann dieser mindestens zwei Substituenten aufweisen, die jeweils ein Sauerstoff-Atom enthalten. Das Sauerstoff-Atom kann dabei direkt am Phenyl-Ring gebunden sein (-O-Phenyl-O-) oder kann in einen Alkylrest eingebaut sein, der an den Phenyl-Ring gebunden ist (z.B. -O-CH₂-Phenyl-CH₂-O-).

Im Falle eines Pyridin-Ringes als Linker L kann dieser mindestens zwei Substituenten aufweisen, die jeweils ein Stickstoff-Atom enthalten. Das Stickstoff-Atom kann dabei direkt am Pyridin-Ring gebunden sein (z.B. -NH-Pyridin-NH-) oder kann in einen Alkylrest eingebaut sein, der an den Pyridin-Ring gebunden ist (z.B. -NH-CH₂-Pyridin-CH₂-NH-). In einer besonders bevorzugten Variante wird als Linker L ein Pyridin-Ring gemäß -NRₓ-Pyridin-NRₓ-verwendet, wobei Rₓ ein C1-C5 Alkyl, bevorzugt ein Methyl, Ethyl oder Propyl, insbesondere ein Ethyl ist.

Die Substituenten R¹, R², R³ des Liganden sind bevorzugt ausgewählt aus einer Gruppe umfassend substituiertes und nicht-substituiertes C₁-C₁₂-Alkyl, substituiertes und nicht-substituiertes Phenyl, substituiertes und nicht-substituiertes C₅-C₆-Heteroaryl, substituiertes und nicht-substituiertes Naphthyl, substituiertes und nicht-substituiertes C₃-C₁₀ Cycloalkyl, substituiertes und nicht-substituiertes C₇-C₁₈ Alkylphenyl, substituiertes und nicht-substituiertes C₅-C₇-Cycloalkenyl, substituiertes und nicht-substituiertes C₂-C₇-Heteroalkylen.

In einer Variante sind die Substituenten R¹, R², R³ des Liganden ausgewählt aus einer Gruppe umfassend Methyl, Ethyl, n-Propyl, Isopropyl, n-Butyl, s-Butyl, t-Butyl, Pentyl, Hexyl, Cyclohexyl, Vinyl, 1-Propenyl, 2-Propenyl, Butenyl oder substituiertes und nicht substituiertes Phenyl.

Dabei sind die Reste t-Butyl, Adamantan und substituiertes und nicht substituiertes Phenyl besonders bevorzugt. Im Falle eines substituierten Phenyls sind die Substituenten bevorzugt ausgewählt aus einer Gruppe umfassend Propyl oder Isopropyl.

In einer besonders bevorzugten Ausführungsvariante weist der NHSi Ligand des vorliegenden Katalysators die folgende Struktur auf:

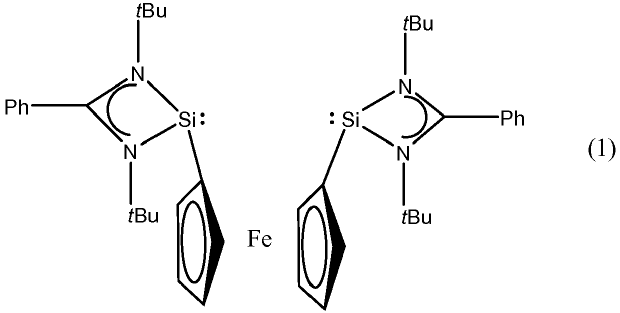

In weiteren bevorzugten Ausführungsform umfasst der NHSi-Ligand des vorliegenden Katalysators eine der folgenden Strukturen:

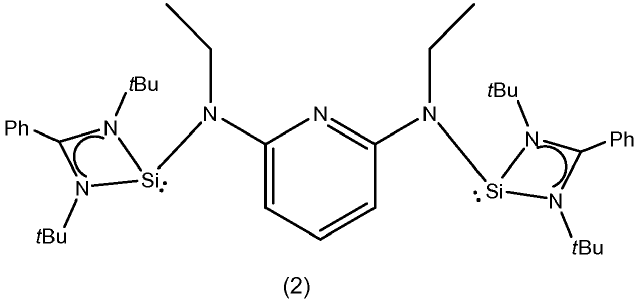

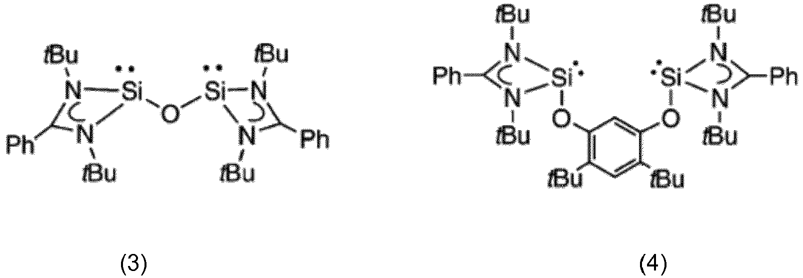

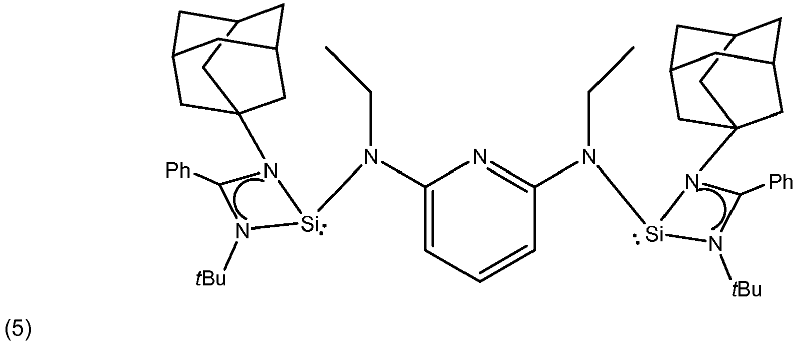

Vorliegend bildet sich bei der Vermischung von Ligand und Metall unter Synthesegasbedingungen bevorzugt ein Komplex in Form eines Metallcarbonylhydrids der allgemeinen Formel (II) aus 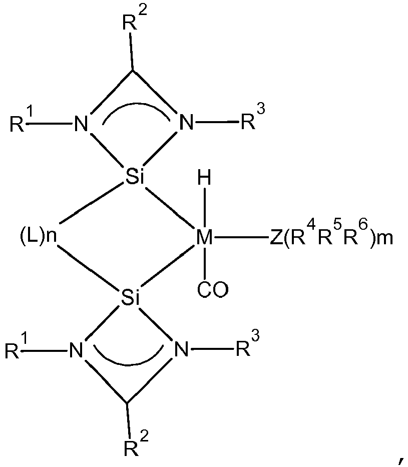 wobei
- L, R¹, R², R³ und M die obige Bedeutung aufweisen,
- Z ein nicht-metallisches Element der Gruppe Va des Periodensystems der Elemente oder ein CO-Ligand ist,
- R⁴, R⁵, R⁶ ausgewählt sind aus der Gruppe umfassend substituiertes und nicht-substituiertes Alkyl, substituiertes und nicht-substituiertes Aryl, substituiertes und nicht-substituiertes Alkenyl, substituiertes und nicht-substituiertes Alkinyl, substituiertes und nicht-substituiertes Cycloalkyl, substituierte und nicht-substituierte Heterocyclen,
- wobei R⁴, R⁵ und R⁶ jeweils gleich oder verschieden voneinander sein können, und
- m = 0-3, bevorzugt 3 ist.

Im Falle des Komplexes der allgemeinen Struktur (II) ist Z insbesondere Phosphor oder Stickstoff, wobei Phosphor besonders bevorzugt ist.

Die Gruppen R⁴, R⁵, R⁶ sind bevorzugt ausgewählt aus der Gruppe umfassend substituiertes und nicht-substituiertes C₁-C₁₂-Alkyl, substituiertes und nicht-substituiertes Phenyl, substituiertes und nicht-substituiertes C₅-C₆-Heteroaryl, substituiertes und nicht-substituiertes Naphthyl, substituiertes und nicht-substituiertes C₃-C₇ Cycloalkyl, substituiertes und nicht-substituiertes C₇-C₁₈ Alkylphenyl, substituiertes und nicht-substituiertes C₅-C₇-Cycloalkenyl, substituiertes und nicht-substituiertes C₂-C₇-Heteroalkylen.

In einer besonders bevorzugten Variante sind die Gruppen R⁴, R⁵, R⁶ ausgewählt aus der Gruppe umfassend substituiertes und nicht-substituiertes Phenyl.

In einer Variante wird der vorliegende Metallcarbonylhydrid-Komplex durch Umsetzung von HRh(CO)(PPh₃)₃ in Analogie zu der folgenden Reaktionsgleichung erhalten:

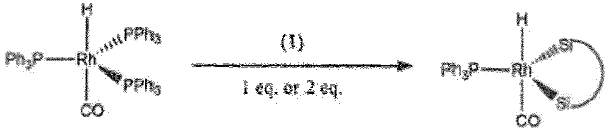

Wie aus der obigen Reaktionsgleichung entnehmbar, werden die Phosphin-Liganden durch den zweizähnigen (bidentaten) Silylen-Liganden substituiert. Die Silylen-Liganden als Chelat-Liganden weisen eine hohe Komplexbildungskonstante auf und können einzähnige Liganden leicht substituieren. Bei der obigen Komplexbildungsreaktion verbleibt ein Phosphin-Ligand am Metall-Zentrum, wodurch die Möglichkeit der Koordination des Olefins an den Silylenmodifizierten Rhodium-Komplex erhöht wird, da der Phosphin-Ligand durch das Olefin substituiert werden kann.

Wie oben erwähnt, können die einzelne Substituenten R1-R6 jeweils substituiert oder nichtsubstituiert vorliegen.

Der Begriff "substituiert" in Verwendung mit "Alkyl", "Alkenyl", "Aryl", etc., bezeichnet die Substitution eines oder mehrerer Atome, in der Regel H-Atome, durch einen oder mehrere der folgenden Substituenten, bevorzugt durch einen oder zwei der folgenden Substituenten: Halogen, Hydroxy, geschütztes Hydroxy, Oxo, geschütztes Oxo, C₃-C₇-Cycloalkyl, bicyclisches Alkyl, Phenyl, Naphtyl, Amino, geschütztes Amino, monosubstituiertes Amino, geschütztes monosubstituiertes Amino, disubstituiertes Amino, Guanidino, geschütztes Guanidino, ein heterozyklischer Ring, ein substituierter heterozyklischer Ring, Imidazolyl, Indolyl, Pyrrolidinyl, C₁-C₁₂-Alkoxy, C₁-C₁₂-Acyl, C₁-C₁₂-Acyloxy, Acryloyloxy, Nitro, Carboxy, geschütztes Carboxy, Carbamoyl, Cyano, Methylsulfonylamino, Thiol, C₁-C₁₀-Alkylthio und C₁-C₁₀-Alkylsulfonyl. Die substituierten Alkygruppen, Arylgruppen, Alkenylgruppen, können einmal oder mehrfach substituiert sein und bevorzugt 1- oder 2-mal, mit denselben oder unterschiedlichen Substituenten.

Der Begriff "C₁-C₁₂-Alkyl" bezeichnet Reste, wie Methyl, Ethyl, n-Propyl, Isopropyl, n-Butyl, Isobutyl, s-Butyl, t-Butyl, Amyl, t-Amyl, Hexyl, Heptyl, Octyl, Nonyl, Decyl, Undecyl, Dodecyl, und ähnliche. Bevorzugte C₁-C₁₂-Alkylgruppen sind Methyl, Ethyl, Isobutyl, s-Butyl und Isopropyl.

Beispiele für die oben substituierten Alkylgruppen schließen 2-Oxo-prop-1-yl, 3-Oxo-but-1-yl, Cyanomethyl, Nitromethyl, Chlormethyl, Hydroxymethyl, Tetrahydropyranyloxymethy, Trityloxymethyl, Propionyloxymethyl, Aminomethyl, Carboxymethyl, Allyloxycarbonylmethyl, Allyloxycarbonylaminomethyl, Methoxymethyl, Ethoxymethyl, t-Butoxymethyl, Acetoxymethyl, Chlormethyl, Brommethyl, Idmethyl, Trifluormethyl, 6-Hydroxyhexyl, 2,4-Dichlor(n-butyl), 2-Aminopropyl, 1-Chlorethyl, 2-Chlorethyl, 1-Bromethyl, 2-Bromethyl, 1-Fluorethyl, 2-Fluorethyl, 1-lodethyl, 2-Iodethyl, 1-Chlorpropyl, 2-Chlorpropyl, 3-Chlorpropyl, 1-Brompropyl, 2-Brompropyl, 3-Brompropyl, 1-Fluorpropyl, 2-Fluorptopyl, 3-Fluorpropyl, 1-Iodpropyl, 2-lodpropyl, 3-Iodpropyl, 2-Aminoethyl, 1-Aminoethyl, N-Benzoyl-2-Aminoethyl, N-Acetyl-2-Aminoethyl, N-Benzoyl-1-Aminoethyl, N-Acetyl-1-Aminoethyl und ähnliche ein.

Beispiele für die oben substituierten Alkenylgruppen schließen Styrolyl, 3-Chlor-propen-1-yl, 3-Chlor-buten-1-yl, 3-Methoxy-propen-2-yl, 3-Phenyl-buten-2-yl, 1-Cyano-buten-3-yl und ähnliche ein. Die Stereoisomerie ist nicht wesentlich und alle Stereoisomere können für ein jeweiliges substituiertes Alkenyl verwendet werden.

Der Begriff "Alkinyl", wie hier verwendet, bezeichnet bevorzugt einen Rest der Formel R-C=C-, insbesondere ein "C₂-C₆-Alkinyl". Beispiele für C₂-C₆-Alkinyle schließen ein: Ethinyl, Propinyl, 2-Butinyl, 2-Pentinyl, 3-Pentinyl, 2-Hexinyl, 3-Hexinyl, 4-Hexinyl, Vinyl sowie Di- und Tri-ine von geraden und verzweigten Alkylketten.

Der Begriff "Aryl", wie hierin verwendet, bezeichnet bevorzugt aromatische Kohlenwasserstoffe, beispielsweise Phenyl, Benzyl, Naphthyl, oder Anthryl. Substituierte Arylgruppen sind Arylgruppen, die, wie oben definiert, mit einem oder mehreren Substituenten substituiert sind.

Der Begriff "Cycloalkyl" umfasst bevorzugt die Gruppen Cyclopropyl, Cyclobutyl, Cyclopentyl, Cyclohexyl,Cycloheptyl und Adamantan.

Der Begriff "Cycloalkenyl" umfasst bevorzugt substituierte oder nicht-substituierte cyclische Gruppen wie Cyclopentenyl oder Cyclohexenyl. Auch werden vom dem Begriff "Cycloalkenyl" cyclische Gruppen mit konjugierten Doppelbindungen wie z.B Cyclohexadiene abgedeckt.

Der Begriff "Alkenyl" umfasst im Sinne der vorliegenden Anmeldungen Gruppen mit einer oder mehreren Doppelbindungen, wobei die Doppelbindungen auch in konjugierter Form vorliegen können, wie z.B. Butadiene.

Der Begriff "Heteroaryl" bedeutet ein heterozyklisches aromatisches Derivat, das ein fünfgliedriges oder sechsgliedriges Ringsystem mit 1-4 Heteroatomen, wie etwa Sauerstoff, Schwefel und/oder Stickstoff hat, insbesondere Stickstoff, entweder alleine oder zusammen mit Schwefel oder Sauerstoff-Ringatomen. Beispiele für Heteroaryle schließen Pyridinyl-, Pyrimidinyl, Pyrazinyl, Pyridazinyl, Pyrrolo, Furano, Oxazolo, Isooxazolo, Phthalimido, Thioazolo und ähnliches ein. Der Begriff "substituiertes Heteroaryl" bezeichnet das oben beschriebene Heteroaryl, welches beispielsweise mit einem oder mehreren und bevorzugt ein oder zwei Substituenten substituiert ist, die wie oben beschrieben sind.

Der Begriff "C₇-C₁₈-Alkylphenyl" bezeichnet eine C₁-C₁₂-Alkylgruppe, die an einer beliebigen Position innerhalb der Alkylkette durch ein Phenylrest substituiert ist. Die Definition schließt die Gruppen der Formel -Phenyl-Alkyl, -Alkyl-Phenyl- und Alkyl-Phenyl-Alkyl- ein. Beispiele für "C₇-C₁₈-Alkylphenyl" schließen Benzyl, 2-Phenylethyl, 3-Phenyl(n-propyl), 4-Phenylhexyl, 3-Phenyl(n-amyl), 3-Phenyl(s-butyl) und ähnliche ein. Bevorzugte C₇-C₁₈-Alkylphenylgruppen sind alle der bevorzugten, hierin beschriebenen Alkylgruppen, in Kombination mit einer Phenylgruppe. Der Begriff "substituiertes C₇-C₁₈-Alkylphenyl" bezeichnet eine C₇-C₁₈-Alkylphenyl-Gruppe, wie oben beschrieben, bei der der Alkylrest und/oder der Phenylrest durch eine der oben als Substituenten definierten Gruppen substituiert ist.

Der Begriff "C₃-C₇-Cycloalkyl" umfasst die Gruppen Cyclopropyl, Cyclobutyl, Cyclopentyl, Cyclohexyl und Cycloheptyl.

Der Begriff "C₅-C₇-Cycloalkenyl" bezeichnet einen 1,2 oder 3-Cyclopentenylring, einen 1,2,3 oder 4-Cyclohexenylring oder einen 1,2,3,4 oder 5-Cycloheptenylring. Die Bezeichnung "substituiertes C₅-C₇-Cycloalkenyl bezeichnet einen C5-C7-Cycloalkenylring, wie oben beschrieben, der durch ein C₁-C₁₂-Alkylrest, Halogen, Hydroxy, geschütztes Hydroxy, C₁-C₁₂-Alkoxy, Trifluormethyl, Carboxy, geschütztes Carboxy, Oxo, geschütztes Oxo, monosubstituiertes Amino, geschütztes monosubstituiertes Amino, disubstituiertes Amino, Phenyl, substituiertes Phenyl, Amino oder geschütztes Amino substituiert ist.

Der Begriff "Alkylen" bezeichnet eine Gruppe -(CRaRb)x-, wobei Ra,Rb H oder eine der oben genannten Substituenten sein kann und x ≥ 1 ist, wobei x = 1, 2 oder 3 bevorzugt sind.

Der Begriff "Heteroalkylen" bezeichnet eine Alkylen-Gruppe, in der mindestens ein C-Atom durch ein Heteroatom wie N oder O ersetzt ist.

Wie bereits mehrfach erwähnt, wird der vorliegende Katalysator zur Hydroformylierung von Olefinen verwendet. Die in der Hydroformylierung verwendeten Olefine sind unter anderem Styrol oder C3-C15 Olefine, bevorzugt C7-C12 Olefine, wie 1-Okten oder 1-Dodecen. Von weiterer industrieller Bedeutung sind auch funktionalisierte Olefine wie Allylalkohole, Alkenylether, Alkenylester oder auch konjugierte Olefine.

Die Hydroformylierung von Olefinen in Gegenwart des (in situ gebildeten) erfindungsgemäßen Katalysators wird bevorzugt bei einem Druck zwischen 10 und 100 bar, bevorzugt zwischen 20 und 70 bar, insbesondere bevorzugt zwischen 30 und 50 bar, und einer Temperatur zwischen 50 und 150°C, bevorzugt zwischen 50 und 100°C durchgeführt.

In einer Ausführungsvariante umfasst die Hydroformylierungsreaktion folgende Schritte:
- Bereitstellen eines Reaktionsgemisches aus mindestens einem NHSi-Liganden und mindestens einem Metall-Precursor in einem geeigneten Lösungsmittel und Zugabe von mindestens einem Substrat zur Hydroformylierung in einem geeigneten Reaktionsreaktor, bevorzugt unter Inertgas-Atomsphäre;
- Zugabe von Synthesegas (aus Kohlenstoffmonoxid und Wasserstoff) zu dem Reaktionsgemisch im Reaktionsreaktor; und
- Durchführung der Hydroformylierungsreaktion bei einer Temperatur zwischen 50 und 100°C und einem Druck zwischen 10 und 50 bar, bevorzugt 30 und 50 bar.

Als Lösungsmittel werden bevorzugt aromatische Lösungsmittel wie Toluol, Benzol oder o, m, p - Xylol verwendet.

In einer noch weitergehenden Variante umfasst die Hydroformylierungsreaktion die Schritte:
- Vorbereitung des Reaktionsgemisches: Die Vorbereitung des Reaktionsgemisches erfolgte unter Standard Schlenk Techniken. Der Metall-Precursor und der entsprechende Ligand wurden eingewogen. Dazu wurde frisch destilliertes Substrat und Lösungsmittel hinzugegeben. In einer Variante wurde als Lösungsmittel Toluol und als Substrat Styrol verwendet.
- Inertisierung des Reaktors: Die Hydroformylierungen wurden in einem 100 mL Edelstahl Autoklaven durchgeführt. Der Reaktor wurde eine Stunde bei 110 °C ausgeheizt und anschließend jeweils 3x evakuiert und mit Stickstoff gespühlt.
- Befüllen des Reaktors mit dem Reaktionsgemisch: Das Reaktionsgemisch wurde mittels einer Spritze unter Stickstoff Gegenstrom bei Reaktionstemperatur eingespritzt.
- Einstellung der Verfahrensbedingungen: Nach der Befüllung des Reaktors wurde der Reaktor geschlossen. Der Reaktor wurde mit Synthesegas befüllt, wobei der Druck 30 bar betrug. Der Verbrauch an Synthesegas wird über einen Massenflussregler kontrolliert, sodass während der Reaktionszeit isobare Bedingungen vorliegen. Die Reaktionstemperatur beträgt bei allen Reaktionen zwischen 50 °C und 100 °C.
- Probennahme und Analyse: Proben zur Bestimmung des Umsatzes und der Katalysatoraktivität wurden in regelmäßigen Zeitabständen genommen, mit Aceton verdünnt und gaschromatographisch untersucht.

In einer Variante des vorliegenden Hydroformylierungsverfahrens wird der Katalysator aus dem Liganden und einem das Metall enthaltenden Vorläuferkomplexes in situ in der Reaktionsmischung gebildet, wobei bevorzugt 3 eq. Ligand und 0.01 mmol Metall verwendet werden.

Die Erfindung wird nachfolgend unter Bezugnahme auf die Figuren an mehreren Ausführungsbeispielen erläutert. Es zeigen:

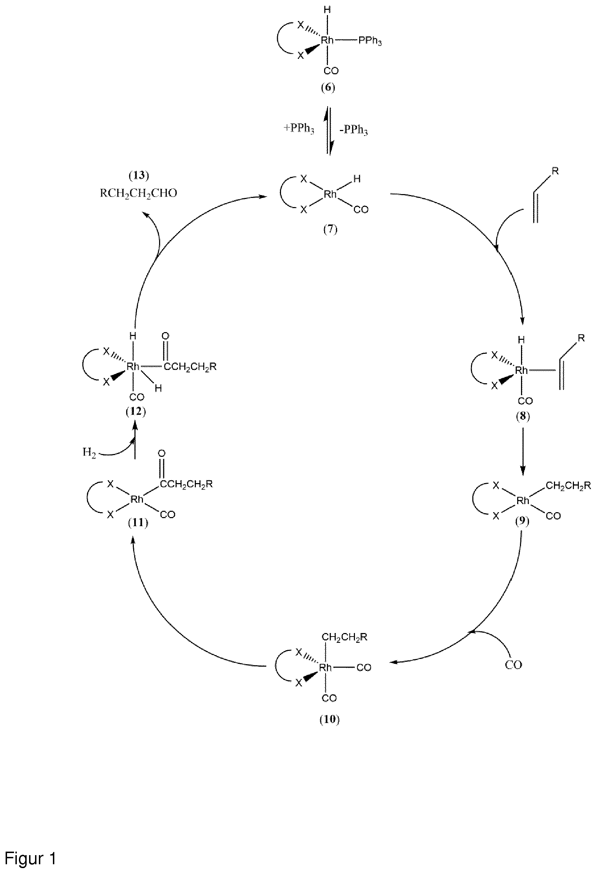

- Figur 1: eine schematische Darstellung des Katalysezyklus einer Rhodium-katalysierten Hydroformylierung.

### Ausführungsbeispiel 1: Herstellen eines ersten NHSi-Liganden (1)

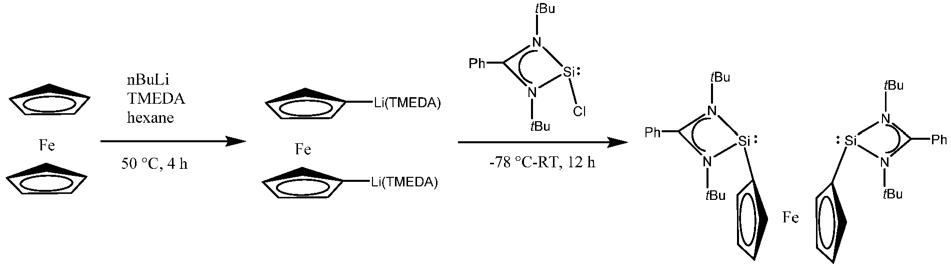

Alle Experimente wurden mit Standard Schlenk Techniken durchgeführt mit trockenem Stickstoff als Inertgas. Zu einer Lösung aus Hexan (10 mL) mit Ferrocen (600 mg, 3,23 mmol) und TMEDA (937 mg, 8,06 mmol) wurde bei 0 °C 1,6 M n-Butyllithium (4,23 mL, 6,77 mmol) gegeben. Die Reaktionslösung wurde bei 50 °C 4 Stunden gerührt. Anschließend wurde die Reaktionslösung auf-78 °C gekühlt. Hierzu wurde eine Lösung aus dem Chlorosilylen (1,9 g, 6,45 mmol) in Toluol (30 mL) über 5 Minuten hinzugetropft. Das Gemisch wurde über Nacht bei Raumtemperatur gerührt, anschließend alle flüchtigen Bestandteile unter Vakuum entfernt und der Rückstand mit Pentan extrahiert. Die tief roten Kristalle von (1) wurden in Pentan bei 0 °C gelagert.
¹H-NMR (400.13 MHz, C₆D₆, 298K, ppm): δ = 1.16 (s, 36 H, NC(C*H*₃)₃), 4.51 (t, ³J (H,H) = 1.5 Hz, 4 H, FeC*H*), 4.72 (t, ³J (H,H) = 1.5 Hz, 4 H, FeC*H*), 6.92- 7.07 (m, 10 H, arom. *H*); ¹³C{¹H} NMR (100.61 MHz, C₆D₆, 298K, ppm) *δ* = 31.8 (NC(*C*H₃)₃), 53.0 (N*C*(CH₃)₃), 70.9 (Fe*C*H), 72.7 (FeCH), 84.6 (Si*C*), 128.9, 129.4, 130.5, 134.9 (arom. *C*), 160.4 (N*C*N); ²⁹Si{¹H} NMR (79.49 MHz, C₆D₆, 298K, ppm) *δ* = 43.3;
Charakterisierung des entsprechenden Rhodium-Komplexes HRh(CO)(PPh₃):

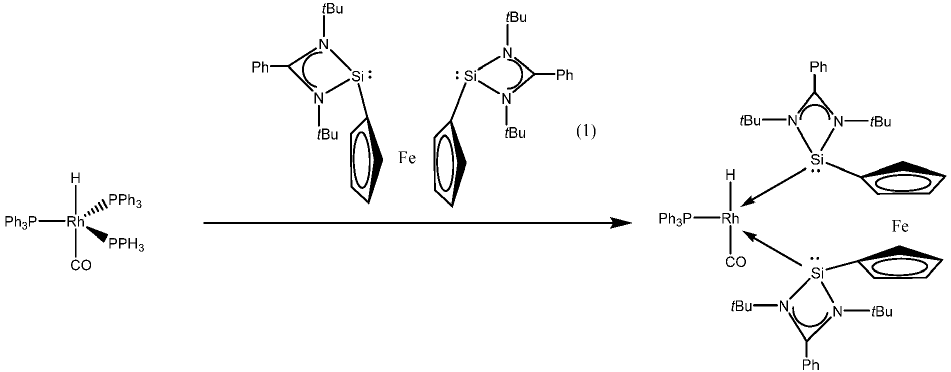

Der Rhodium-Precursor Tris(triphenylphosphin)hydridocarbonyl-Rhodium (I) HRh(CO)(PPh₃)₃ und der NHSi Ligand **(1)** wurden in äquimolaren Verhältnis in 0,5 mL C₆D₆ gelöst. Eine Orangefärbung wurde unverzüglich festgestellt. NMR Ergebnisse belegen die Bildung des Rhodium-Komplexes HRh(CO)(PPh₃)**(1)**.
¹H-NMR (200 MHz, THF-d₈, 298 K, ppm): δ = -9.43 (ps t: 1H, ¹*J* (H,Rh) and ²*J* (H,P) = 11.4 Hz) (P Kopplung sichtbar), 0.87 (s, 18 H, 2 x *H*-*^{t}*Bu-N), 1.29 (s, 18 H, 2 x *H*-*^{t}*Bu-N), 4.15 (m, 8 H, 4 x *H*-Ferrocene), 7.03-7.70 (m, 55 H, *H*-PPh₃ + m, 10 H, *H*-Ph). ³¹P-NMR (81 MHz, THF, 298 K, ppm): δ = -5.4 (s, *P*-PPh₃) (Freier Ligand), 44.7 (d, ¹*J* (P,Rh) = 98.7 Hz).

Bei Zugabe eines Überschusses des NHSi Liganden wurde ebenfalls der Rhodium-Komplex HRh(CO)(PPh₃) gebildet, wobei überschüssiger Ligand nicht umgesetzt wurde.

### Ausführungsbeispiel 2: Herstellen eines zweiten NHSi Liganden (2)

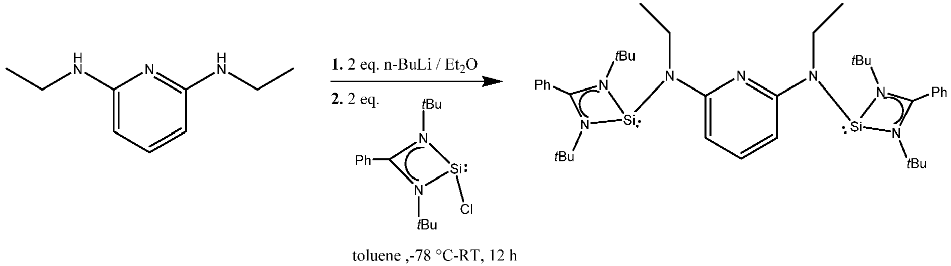

¹H-NMR (400.13 MHz, C₆D₆, 298 K, ppm): symmetrisches Konformer: δ = 1.16 (s, 36H, NC(CH₃)₃), 1.63 (t, ³J (H,H) = 6.9 Hz, 6 H, NCH₂-CH₃), 3.77 (q, ³J (H,H) = 6.9 Hz, 4 H, NCH₂-CH₃), 6.87-7.09 (m, 10 H, arom. C-H), 7.34-7.50 (m, 3 H, arom. C-H py.). asymmetrisches Konformer: δ = 1.14 (s, 36H, NC(CH₃)₃), 1.55 und 1.68 (t, ³J (H,H) = 6.9 Hz, 6 H, NCH₂-CH₃), 3.71 und 4.62 (q, ³J (H,H) = 6.9 Hz, 4 H, NCH₂-CH₃), ¹³C{¹H}-NMR (100,61 MHz, C₆D₆, 298 K, ppm): symmetrisches Konformer: δ = 16.9 (NCH₂-CH₃), 31.6 (NC(CH₃)₃) 31.9 (NCH₂-CH₃), 52.9 (NC(CH₃)₃), 101.8 (3,5-Cₐᵣₒₘ. py), 127.6 (Cₐᵣₒₘ), 128.5 (Cₐᵣₒₘ), 129.3 (Cₐᵣₒₘ), 129.3 (Cₐᵣₒₘ), 129.4 (Cₐᵣₒₘ), 130.0 (Cₐᵣₒₘ), 130.5 (Cₐᵣₒₘ), 130.5 (Cₐᵣₒₘ), 134.7 (Cₐᵣₒₘ quaternäres Ph), 136.9 (4-Cₐᵣₒₘ py), 161.2 (2,6-Cₐᵣₒₘ py), 161.4 (NCN). asymmetrisches Konformer: δ = 18.0 und 16.0 (NCH₂-CH₃), 31.4 und 31.5 (NC(CH₃)₃), 36.8 und 43.9 (NCH₂-CH₃), 53.3 (NC(CH₃)₃), 103.0 und 103.9 (3,5-Cₐᵣₒₘ. py), 134.0 und 134.5 (Cₐᵣₒₘ quaternäres Ph), 136.4 (4-Cₐᵣₒₘ py). ²⁹Si{¹H}-NMR (79,49 MHz, C₆D₆, 298 K, ppm): symmetrisches Konformer: δ = -14.9. asymmetrisches Konformer: δ = -13.8 und -17.1.

### Ausführunsgbeispiel 3: Hydroformylierung

### Vorbereitung des Reaktionsgemisches:

In einem 100 mL Schlenkkolben wurde der Rhodium-Precursor HRh(CO)(PPh₃)₃ (0,01 mmol, 9,188 mg, 1 eq.) und der NHSi-Ligand (1) (3 eq.) vorgelegt und in frisch destillierten Toluol (0,434 mol, 40,0 g) gelöst. Anschließend wurde frisch destilliertes Styrol (0,038 mol, 4,0 g, 3800 eq.) hinzugegeben.

### Experimentelle Durchführung für die Hydroformylierung von Styrol:

In einem 100 mL Edelstahl Autoklaven wurde die Hydroformylierung durchgeführt. Vor Zugabe des Reaktionsgemisches wurde der Reaktor bei 110 °C für eine Stunde ausgeheizt und anschließend jeweils 3x evakuiert und mit Stickstoff gespühlt. Nach Abkühlung auf Reaktionstemperatur wurde das Reaktionsgemisch mittels einer Spritze unter Stickstoff Gegenstrom in den Reaktor injiziert. Anschließend wurde dem Reaktor ein Reaktionsdruck von 30 bar Synthesegas (1:1 Wasserstoff und Kohlenstoffmonoxid) bei einer Rührgeschwindigkeit von 200 rpm aufgeprägt und nach Erreichen des Reaktionsdruckes die Rührgeschwindigkeit auf 1200 rpm erhöht. Um isobare Reaktionsbedingungen zu erreichen wurde umgesetztes Synthesegas mittels eines Massenflussreglers hinzudosiert. Proben wurden mit Aceton verdünnt und gaschromatografisch analysiert.

Figur 1 illustriert einen katalytischen Zyklus eines Rhodium-Katalysators mit einem zweizähnigen Liganden. Beginnend mit der trigonalen-bipyramidalen Hydrid-Spezies 6, welche in situ gemäß obiger Reaktionsgleichung erzeugt wird. Die Bildung des aktiven Komplexes 7 wird durch den Abgang eines Triphenylphophin-Liganden initiiert. Die π-Koordination des Alkens an den ungesättigten Rhodium-Komplex 7 führt zur Bildung des Komplexes 8, der durch Wanderung des Hydrids zum korrespondierenden Rhodium-Alkyl-Komplex 9. Nach Koordination eines zusätzlichen CO-Moleküls unter Ausbildung des Komplexes 10 wird CO in die Rhodium-Alkyl-Bindung unter Ausbildung des Rhodium-Acyl-Komplexes 11 eingeführt. Durch die Zugabe von Wasserstoff, was zur Bildung des Komplexes 12 führt wird der Aldehyd 12 abgetrennt und des aktive Rhodium-Hydrid Komplex 7 wird regeneriert.

Zur Bewertung der Katalysatoraktivität mit dem erfindungsgemäßen zweizähnigen Liganden (in der Tabelle mit **(1)** bezeichnet) wurde die Hydroformylierung von Styrol im Vergleich zu einem häufig verwendeten zweizähnigen Phosphinliganden XantPhos untersucht. Hierbei wurde als Kennzahl die "turn over frequency" (TOF) verwendet, welche die Anzahl der katalytischen Zyklen des Katalysators pro Zeiteinheit beschreibt. Die Ergebnisse sind in Tabelle 1 für verschiedene Temperaturen gezeigt.

**Tabelle 1: TOF-Werte für die Hydroformylierung von Styrol mit zweizähnigen Liganden bei verschiedenen Temperaturen. Reaktionsbedingungen: 40 g Toluol, 4 g Styrol, n_{Rh} 0,01 mmol, n_{ligand} = 3 eq, p = 30 bar, rpm = 1200. TOF bestimmt nach 60 min bei 50°C, nach 30 min bei 80°C und bei 100°C nach 10 min.**

| **Ligand** | **Temperatur T[°C]** | **TOF³ [1/h]** |
|---|---|---|
| (1) | 50 | 83 |
| XantPhos | 50 | 32 |
| (1) | 80 | 2621 |
| XantPhos | 80 | 646 |
| (1) | 100 | 9075 |
| XantPhos | 100 | 3007 |

Wie aus Tabelle 1 ersichtlich wurde die TOF bei allen Temperaturen bei Verwendung des erfindungsgemäßen zweizähnigen NHSi Liganden **(1)** gegenüber XantPhos verdreifacht, was auf eine deutliche Aktivitätssteigerung hinweist. Auch bleibt der Katalysator bei hohen Temperaturen stabil.

## Patentansprüche

1. Katalysator für die Hydroformylierung von mindestens einem Olefin
umfassend
einen Liganden der allgemeinen Formel (I) 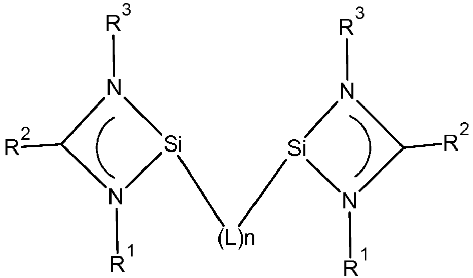 wobei
- R¹, R², R³ ausgewählt sind aus der Gruppe umfassend substituiertes und nicht-substituiertes Alkyl, substituiertes und nicht-substituiertes Aryl, substituiertes und nicht-substituiertes Alkenyl, substituiertes und nicht-substituiertes Alkinyl, substituiertes und nicht-substituiertes Cycloalkyl, substituierte und nicht-substituierte Heterocyclen,
- wobei R¹, R² und R³ jeweils gleich oder verschieden voneinander sein können,
- L ausgewählt ist aus einer Gruppe umfassend einen Sandwich-Komplex, eine Sauerstoffgruppe, substituiertes und nicht-substituiertes Alkylen oder Heteroalkylen, substituiertes Aryl oder Heteroaryl, wobei Aryl und Heteroaryl jeweils mit mindestens zwei Heteroatome enthaltenden Gruppen substituiert sind und über die mindestens zwei Heteroatome der Substituenten mit dem Si gekoppelt sind,
- n = 1-10, bevorzugt 1-5, insbesondere bevorzugt 1, 2 oder 3 ist; und
- wobei der Ligand über die Si-Gruppe mit dem Metall M aus der Gruppe Vlllb des Periodensystems der Elemente gekoppelt ist.

2. Katalysator nach Anspruch 1, **dadurch gekennzeichnet, dass** M Co oder Rh ist.

3. Katalysator nach Anspruch 1 oder 2, **dadurch gekennzeichnet, dass** L als ein Sandwich-Komplex in Form eines Metallocen-Komplexes, insbesondere Ferrocen, vorliegt.

4. Katalysator nach Anspruch 1 oder 2, **dadurch gekennzeichnet, dass** L in Form eines Phenyl- oder Pyridin-Ringes vorliegt, wobei der Phenyl- oder Pyridin-Ring jeweils mit mindestens zwei Heteroatome, bevorzugt Sauerstoff oder Stickstoff, enthaltenden Gruppen substituiert sind.

5. Katalysator nach einem der vorhergehenden Ansprüche, **dadurch gekennzeichnet, dass** R¹, R², R³ ausgewählt sind aus einer Gruppe umfassend substituiertes und nicht-substituiertes C₁-C₁₂-Alkyl, substituiertes und nicht-substituiertes Phenyl, substituiertes und nicht-substituiertes C₅-C₆-Heteroaryl, substituiertes und nicht-substituiertes Naphthyl, substituiertes und nicht-substituiertes C₃-C₁₀ Cycloalkyl, substituiertes und nicht-substituiertes C₇-C₁₈ Alkylphenyl, substituiertes und nicht-substituiertes C₅-C₇-Cycloalkenyl, substituiertes und nicht-substituiertes C₂-C₇-Heteroalkylen.

6. Katalysator nach einem der vorhergehenden Ansprüche, **dadurch gekennzeichnet, dass** R¹, R², R³ ausgewählt sind aus einer Gruppe umfassend Methyl, Ethyl, n-Propyl, Isopropyl, n-Butyl, s-Butyl, t-Butyl, Pentyl, Hexyl, Cyclohexyl, Vinyl, 1-Propenyl, 2-Propenyl, Butenyl, Adamantan oder Phenyl.

7. Katalysator nach einem der vorhergehenden Ansprüche, **dadurch gekennzeichnet, dass** R¹, R², R³ ausgewählt sind aus einer Gruppe umfassend t-Butyl, Adamantan und substituiertes und nicht substituiertes Phenyl.

8. Katalysator nach einem der vorhergehenden Ansprüche, **dadurch gekennzeichnet, dass** Ligand und Metall eine Komplex der allgemeinen Formel (II) 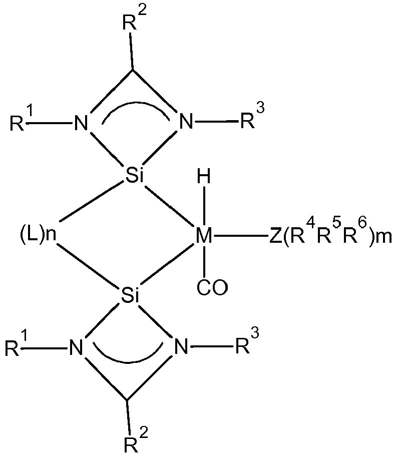 bilden, wobei
- L, R¹, R², R³ und M die obige Bedeutung aufweisen,
- Z ein nicht-metallisches Element der Gruppe Va des Periodensystems der Elemente oder ein CO-Ligand ist,
- R⁴, R⁵, R⁶ ausgewählt sind aus der Gruppe umfassend substituiertes und nicht-substituiertes Alkyl, substituiertes und nicht-substituiertes Aryl, substituiertes und nicht-substituiertes Alkenyl, substituiertes und nicht-substituiertes Alkinyl, substituiertes und nicht-substituiertes Cycloalkyl, substituierte und nicht-substituierte Heterocyclen,
- wobei R⁴, R⁵ und R⁶ jeweils gleich oder verschieden voneinander sein können, und
- m = 0- 3, bevorzugt 3 ist.

9. Katalysator nach Anspruch 8, **dadurch gekennzeichnet, dass** Z Phosphor oder Stickstoff ist.

10. Katalysator nach einem der Ansprüche 8 und 9, **dadurch gekennzeichnet, dass** R⁴, R⁵, R⁶ ausgewählt sind aus der Gruppe umfassend substituiertes und nicht-substituiertes C₁-C₁₂-Alkyl, substituiertes und nicht-substituiertes Phenyl, substituiertes und nicht-substituiertes C₅-C₆-Heteroaryl, substituiertes und nicht-substituiertes Naphthyl, substituiertes und nicht-substituiertes C₃-C₇ Cycloalkyl, substituiertes und nicht-substituiertes C₇-C₁₈ Alkylphenyl, substituiertes und nicht-substituiertes C₅-C₇-Cycloalkenyl, substituiertes und nicht-substituiertes C₂-C₇-Heteroalkylen.

11. Katalysator nach einem der Ansprüche 8 bis 10, **dadurch gekennzeichnet, dass** R⁴, R⁵, R⁶ ausgewählt sind aus der Gruppe umfassend substituiertes und nicht-substituiertes Phenyl.

12. Verwendung eines Katalysators nach einem der vorhergehenden Ansprüche zur Hydroformylierung von Olefinen.

13. Verwendung nach Anspruch 12 zur Hydroformylierung von Styrol und C3-C15 Olefinen, bevorzugt C7-C12 Olefinen wie 1-Okten oder 1-Dodecen.

14. Verfahren zur Hydroformylierung von Olefinen in Gegenwart eines Katalysators nach einem der Ansprüche 1 bis 11 umfassend die Schritte:
- Bereitstellen eines Reaktionsgemisches aus mindestens einem NiHs-Liganden nach einem der Ansprüche 1-6 und mindestens einem Metall-Precursor in einem geeigneten Lösungsmittel und Zugabe von mindestens einem Substrat zur Hydroformylierung in einem geeigneten Reaktionsreaktor, bevorzugt unter Inertgas-Atomsphäre;
- Zugabe von Synthesegas aus Kohlenstoffmonoxid und Wasserstoff zu dem Reaktionsgemisch im Reaktionsreaktor; und
- Durchführung der Hydroformylierungsreaktion bei einer Temperatur zwischen 50 und 100°C und einem Druck zwischen 30 und 50 bar.

15. Verfahren nach Anspruch 14, **dadurch gekennzeichnet, dass** der Katalysator nach einem der Ansprüche 1 bis 11 aus dem Liganden und einem das Metall enthaltenden Vorläuferkomplexes in situ in der Reaktionsmischung gebildet wird.

## Claims

1. Catalyst for the hydroformylation of at least one olefin
comprising
a ligand of general formula (I) 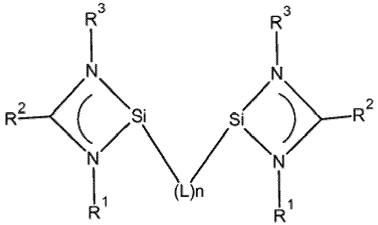 wherein
- R¹, R² and R³ are selected from the group comprising substituted and unsubstituted alkyl, substituted and unsubstituted aryl, substituted and unsubstituted alkenyl, substituted and unsubstituted alkinyl, substituted and unsubstituted cycloalkyl, substituted and unsubstituted heterocycles,
- wherein R¹, R² and R³ can each be the same or different,
- L is selected from a group comprising a sandwich complex, an oxygen group, substituted and unsubstituted alkylene or heteroalkylene, substituted aryl or heteroaryl, wherein aryl and heteroaryl are each substituted with groups comprising at least two heteroatoms and are coupled to the Si via the at least two heteroatoms of the substituents,
- n = 1-10, preferably 1-5, particularly preferably 1, 2 or 3; and
- wherein the ligand is coupled to the metal M from group VIIIb of the periodic table of elements via the Si group.

2. Catalyst according to Claim 1, **characterized in that** M is Co or Rh.

3. Catalyst according to Claim 1 or 2, **characterized in that** L is present as a sandwich complex in the form of a metallocene complex, in particular ferrocene.

4. Catalyst according to Claim 1 or 2, **characterized in that** L is present in the form of a phenyl or pyridine ring, wherein the phenyl or pyridine ring respectively are substituted with groups comprising at least two heteroatoms, preferably oxygen or nitrogen.

5. Catalyst according to one of the preceding claims, **characterized in that** R¹, R² and R³ are selected from a group comprising substituted and unsubstituted C₁-C₁₂ alkyl, substituted and unsubstituted phenyl, substituted and unsubstituted C₅-C₆ heteroaryl, substituted and unsubstituted naphthyl, substituted and unsubstituted C₃-C₁₀ cycloalkyl, substituted and unsubstituted C₇-C₁₈ alkylphenyl, substituted and unsubstituted C₅-C₇ cycloalkenyl, substituted and unsubstituted C₂-C₇ heteroalkylene.

6. Catalyst according to one of the preceding claims, **characterized in that** R¹, R² and R³ are selected from a group comprising methyl, ethyl, n-propyl, isopropyl, n-butyl, s-butyl, t-butyl, pentyl, hexyl, cyclohexyl, vinyl, 1-propenyl, 2-propenyl, butenyl, adamantane or phenyl.

7. Catalyst according to one of the preceding claims, **characterized in that** R¹, R² and R³ are selected from a group comprising t-butyl, adamantane and substituted and unsubstituted phenyl.

8. Catalyst according to one of the preceding claims, **characterized in that** ligand and metal form a complex of general formula (II) 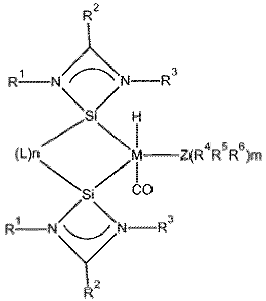 wherein
- L, R¹, R², R³ and M have the above meaning,
- Z is a non-metallic element of group Va of the periodic table of elements or a CO ligand,
- R⁴, R⁵ and R⁶ are selected from the group comprising substituted and unsubstituted alkyl, substituted and unsubstituted aryl, substituted and unsubstituted alkenyl, substituted and unsubstituted alkinyl, substituted and unsubstituted cycloalkyl, substituted and unsubstituted heterocycles,
- wherein R⁴, R⁵ and R⁶ can each be the same or different, and
- m = 0-3, preferably 3.

9. Catalyst according to Claim 8, **characterized in that** Z is phosphorus or nitrogen.

10. Catalyst according to either of Claims 8 and 9, **characterized in that** R⁴, R⁵ and R⁶ are selected from the group comprising substituted and unsubstituted C₁-C₁₂ alkyl, substituted and unsubstituted phenyl, substituted and unsubstituted C₅-C₆ heteroaryl, substituted and unsubstituted naphthyl, substituted and unsubstituted C₃-C₇ cycloalkyl, substituted and unsubstituted C₇-C₁₈ alkylphenyl, substituted and unsubstituted C₅-C₇ cycloalkenyl, and substituted and unsubstituted C₂-C₇ heteroalkylene.

11. Catalyst according to one of Claims 8 to 10, **characterized in that** R⁴, R⁵ and R⁶ are selected from the group comprising substituted and unsubstituted phenyl.

12. Use of a catalyst according to one of the preceding claims for the hydroformylation of olefins.

13. Use according to Claim 12 for the hydroformylation of styrene and C3-C15 olefins, preferably C7-C12 olefins such as 1-octene or 1-dodecene.

14. Method for the hydroformylation of olefins in the presence of a catalyst according to one of Claims 1 to 11 comprising the following steps:
- preparation of a reaction mixture of at least one NiHs ligand according to one of Claims 1-6 and at least one metal precursor in a suitable solvent and addition of at least one substrate for the hydroformylation in a suitable reactor, preferably under an inert gas atmosphere;
- addition of synthesis gas of carbon monoxide and hydrogen to the reaction mixture in the reactor; and
- carrying out the hydroformylation reaction at a temperature of between 50 and 100°C and a pressure of between 30 and 50 bar.

15. Method according to Claim 14, **characterized in that** the catalyst according to one of Claims 1 to 11 is formed from the ligand and a precursor complex comprising the metal in situ in the reaction mixture.

## Revendications

1. Catalyseur pour l'hydroformylation d'au moins une oléfine
comprenant
un ligand de formule générale (I) 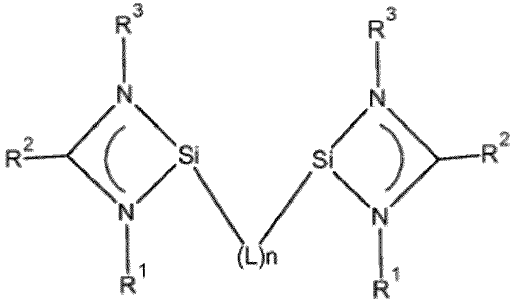
- R¹, R², R³ étant choisis dans le groupe comprenant alkyle substitué et non substitué, aryle substitué et non substitué, alcényle substitué et non substitué, alcynyle substitué et non substitué, cycloalkyle substitué et non substitué, hétérocycles substitués et non substitués,
- ou R¹, R², R³ pouvant à chaque fois être identiques ou différents les uns des autres,
- L étant choisi dans un groupe comprenant un complexe sandwich, un groupe oxygène, alkylène ou hétéroalkylène substitué et non substitué, aryle ou hétéroaryle substitué, ou aryle et hétéroaryle étant à chaque fois substitués par des groupes contenant au moins deux hétéroatomes et étant couplés avec le Si via des au moins deux hétéroatomes des substituants,
- n = 1 à 10, préférablement étant 1 à 5, en particulier préférablement étant 1, 2 ou 3; et ou le ligand étant couplé au métal M du groupe VIIIb du système périodique des éléments via le groupe Si.

2. Catalyseur selon la revendication 1, **caractérisé en ce que** M est Co ou Rh.

3. Catalyseur selon la revendication 1 ou 2, **caractérisé en ce que** L est présent en tant que complexe sandwich sous la forme d'un complexe de métallocène, en particulier de ferrocène.

4. Catalyseur selon la revendication 1 ou 2, **caractérisé en ce que** L est présent sous la forme d'un cycle phényle ou pyridine, ou le cycle phényle ou pyridine étant respectivement substitué par des groupes contenant au moins deux hétéroatomes, préférablement oxygène ou azote.

5. Catalyseur selon l'une quelconque des revendications précédentes, **caractérisé en ce que** R¹, R², R³ sont choisis dans un groupe comprenant C₁₋₁₂-alkyle substitué et non substitué, phényle substitué et non substitué, C₅₋₆-hétéroaryle substitué et non substitué, naphtyle substitué et non substitué, C₃₋₁₀-cycloalkyle substitué et non substitué, C₇₋₁₈-alkylphényle substitué et non substitué, C₅₋₇-cycloalcényle substitué et non substitué, C₂₋₇-hétéroalkylène substitué et non substitué.

6. Catalyseur selon l'une quelconque des revendications précédentes, **caractérisé en ce que** R¹, R², R³ sont choisis dans un groupe comprenant méthyle, éthyle, n-propyle, isopropyle, n-butyle, s-butyle, t-butyle, pentyle, hexyle, cyclohexyle, vinyle, 1-propényle, 2-propényle, butényle, adamantane et phényle.

7. Catalyseur selon l'une quelconque des revendications précédentes, **caractérisé en ce que** R¹, R², R³ sont choisis dans un groupe comprenant t-butyle, adamantane et phényle substitué et non substitué.

8. Catalyseur selon l'une quelconque des revendications précédentes, **caractérisé en ce que** le ligand et le métal forment un complexe de formule générale (II) 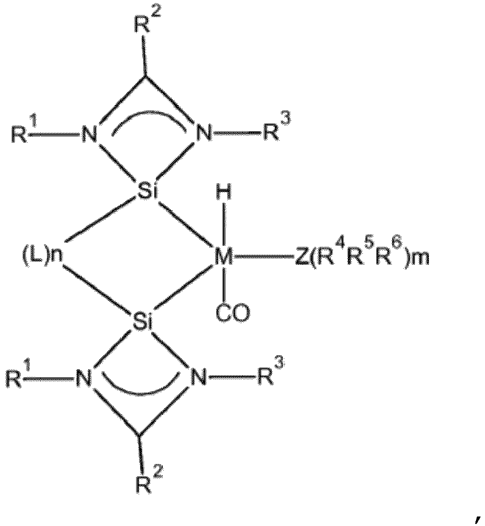
- L, R¹, R², R³ et M présentant la signification ci-dessus,
- Z étant un élément non métallique du groupe Va du système périodique des éléments ou un ligand CO,
- R⁴, R⁵, R⁶ étant choisis dans le groupe comprenant alkyle substitué et non substitué, aryle substitué et non substitué, alcényle substitué et non substitué, alcynyle substitué et non substitué, cycloalkyle substitué et non substitué, hétérocycles substitués et non substitués,
- ou R⁴, R⁵ et R⁶ pouvant être à chaque fois identiques ou différents les uns des autres, et
- m = 0 à 3, préférablement étant 3.

9. Catalyseur selon la revendication 8, **caractérisé en ce que** Z est phosphore ou azote.

10. Catalyseur selon l'une quelconque des revendications 8 et 9, **caractérisé en ce que** R⁴, R⁵, R⁶ sont choisis dans le groupe comprenant C₁₋₁₂-alkyle substitué et non substitué, phényle substitué et non substitué, C₅₋₆-hétéroaryle substitué et non substitué, naphtyle substitué et non substitué, C₃₋₇-cycloalkyle substitué et non substitué, C₇₋₁₈-alkylphényle substitué et non substitué, C₅₋₇-cycloalcényle substitué et non substitué, C₂₋₇-hétéroalkylène substitué et non substitué.

11. Catalyseur selon l'une quelconque des revendications 8 à 10, **caractérisé en ce que** R⁴, R⁵, R⁶ sont choisis dans le groupe comprenant phényle substitué et non substitué.

12. Utilisation d'un catalyseur selon l'une quelconque des revendications précédentes pour l'hydroformylation d'oléfines.

13. Utilisation selon la revendication 12 pour l'hydroformylation de styrène et d'oléfines en C₃₋₁₅, préférablement d'oléfines en C₇₋₁₂ comme le 1-octène ou le 1-dodécène.

14. Procédé pour l'hydroformylation d'oléfines en présence d'un catalyseur selon l'une quelconque des revendications 1 à 11 comprenant les étapes:
- mise à disposition d'un mélange de réaction composé d'au moins un ligand NiHs selon l'une quelconque des revendications 1 à 6 et d'au moins un précurseur métallique dans un solvant approprié et ajout d'au moins un substrat pour l'hydroformylation dans un réacteur de réaction approprié, préférablement sous atmosphère de gaz inerte;
- ajout de gaz de synthèse composé de monoxyde de carbone et d'hydrogène au mélange de réaction dans le réacteur de réaction; et
- mise en œuvre de la réaction d'hydroformylation à une température comprise entre 50 et 100 ° C et une pression comprise entre 30 et 50 bars.

15. Procédé selon la revendication 14, **caractérisé en ce que** le catalyseur selon l'une quelconque des revendications 1 à 11 est formé in situ dans le mélange de réaction à partir du ligand et d'un complexe précurseur contenant le métal.
